(19) **Europäisches Patentamt**
**European Patent Office**
**Office européen des brevets**

(11) **EP 3 417 954 B1**

(12) **EUROPEAN PATENT SPECIFICATION**

(45) Date of publication and mention
of the grant of the patent:
**23.12.2020 Bulletin 2020/52**

(51) Int Cl.:
*B09B 5/00* $^{(2006.01)}$  *A61L 2/03* $^{(2006.01)}$
*A61L 2/04* $^{(2006.01)}$  *A61L 2/10* $^{(2006.01)}$
*A61L 2/18* $^{(2006.01)}$  *A61L 2/20* $^{(2006.01)}$
*B09B 3/00* $^{(2006.01)}$  *C02F 1/58* $^{(2006.01)}$
*C02F 3/34* $^{(2006.01)}$  *H01M 8/16* $^{(2006.01)}$

(21) Application number: **17782071.9**

(22) Date of filing: **12.01.2017**

(86) International application number:
**PCT/JP2017/000862**

(87) International publication number:
**WO 2017/179252 (19.10.2017 Gazette 2017/42)**

(54) **METHOD FOR RECOVERING PULP FIBERS FROM USED ABSORBENT ARTICLES**

VERFAHREN ZUR RÜCKGEWINNUNG VON ZELLSTOFFFASERN AUS GEBRAUCHTEN ABSORBIERENDEN ARTIKELN

PROCÉDÉ DE RÉCUPÉRATION DES FIBRES DE PÂTE À PARTIR D'ARTICLES ABSORBANTS USAGÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.04.2016 JP 2016079179**

(43) Date of publication of application:
**26.12.2018 Bulletin 2018/52**

(73) Proprietor: **Unicharm Corporation**
**Ehime 799-0111 (JP)**

(72) Inventors:
• **KONISHI, Takayoshi**
**Kanonji-shi**
**Kagawa 769-1602 (JP)**
• **HIRAOKA, Toshio**
**Kanonji-shi**
**Kagawa 769-1602 (JP)**
• **YAMAKI, Koichi**
**Kanonji-shi**
**Kagawa 769-1602 (JP)**
• **KAMEDA, Noritomo**
**Kanonji-shi**
**Kagawa 769-1602 (JP)**

(74) Representative: **Dolleymores**
**9 Rickmansworth Road**
**Watford, Hertfordshire WD18 0JU (GB)**

(56) References cited:
**WO-A1-2015/190140    JP-A- 2010 084 031**
**JP-A- 2013 197 047    JP-A- 2015 134 845**
**JP-A- 2015 134 845    JP-A- 2015 182 246**

• **HANAA GADALLAH ET AL:** "Study of Electro De-swelling Properties of Super Absorbents Polymers 1", AUSTRALIAN JOURNAL OF BASIC AND APPLIED SCIENCES, vol. 6, 1 January 2012 (2012-01-01), pages 282-291, XP055555771,
• **Zohuriaan-Mehr ET AL:** "Superabsorbent Polymer Materials: A Review", Iranian Polymer Journal, vol. 17, no. 6, 1 January 2008 (2008-01-01), pages 451-477, XP055058280,
• **Auda Braihi:** "Super absorbent Polymers", , 1 December 2016 (2016-12-01), XP055652338, Retrieved from the Internet: URL:https://www.researchgate.net/publicati on/312021797_Super_absorbent_Polymers [retrieved on 2019-12-12]

**Description**

FIELD

The present invention relates to a method of recovering pulp fibers from a used absorbent article which includes the pulp fibers and super absorbent polymers, such as a paper diaper, etc. More specifically, the present invention relates to a recovering method of pulp fibers from a used absorbent article with small damages to the recovered pulp fibers.

BACKGROUND

An absorbent article, such as a paper diaper, etc., is normally composed of an absorbent body which includes pulp fibers and super absorbent polymers, and an outer wrapping body which covers the absorbent body, and is made of a nonwoven fabric or a plastic film. Such an absorbent article is discarded and incinerated after being used, however, in recent years, in consideration of the environmental aspect, it has been considered to recover and recycle the materials which configure an absorbent article.

Japanese Unexamined Patent Publication No. 2010-84031 discloses a treatment method of a used paper diaper which disinfects and processes the used paper diaper, characterized by including: throwing lime, hypochlorite, and the used paper diaper in a treatment tank, stirring the same for a predetermined period of time while supplying minimum amount of water capable of stirring the same in the treatment tank, discharging liquid in the treatment tank to an outside of the treatment tank and dehydrating the same, and recovering discharged waste water so as to be subjected to water quality treatment and to be discarded.

EP3156541A1 discloses a method for manufacturing recycled pulp by recovering pulp fiber from used sanitary products containing pulp fibers and super absorbent polymer. JP2015134845A relates to a spin-drying/dehydration demineralization method for used super absorbent polymer contained in a paper diaper.

HANAA GADALLAH ET AL: "Study of Electro De-swelling Properties of Super Absorbents Polymers", AUSTRALIAN JOURNAL OF BASIC AND APPLIED SCIENCES, vol. 6, 1 January 2012 (2012-01-01), pages 282-291, investigates the water deswelling properties of the super absorbent polymer poly(acrylic acid sodium salt).

DISCLOSURE OF THE INVENTION

[PROBLEMS TO BE SOLVED BY THE INVENTION]

In the method disclosed in Japanese Unexamined Patent Publication No. 2010-84031, sufficient amount of lime is thrown in for inactivating the super absorbent polymers, and ozone or chlorine compound is used as the disinfecting agent (the sterilizing agent). Accordingly, not only the inside of the treatment tank is to be under high pH (12.4) environment by lime, and pulp fibers are to be converted to alkali cellulose and deteriorated, but also since ozone or chlorine compound is used for sterilization, there may be cases in which the pulp fibers are damaged, the deterioration progresses by repeating recycling, and the pulp fibers are deteriorated to a level that recycling is no longer possible.

The present invention provides a method of efficiently recovering pulp fibers which does not deteriorate the property of the pulp fibers with high safety.

[MEANS FOR SOLVING THE PROBLEMS]

The inventors focused on inactivating the super absorbent polymers by an ion exchange between base dissociated ions (Na+) of the super absorbent polymers and hydrogen ions generated by electrolysis of water, by an electric field formed between electrodes, as well as enabling destruction and sterilization of a cell membrane by an electric field energy, whereby completed the present invention.

The present invention is a method of recovering pulp fibers from a used absorbent article which includes the pulp fibers and super absorbent polymers, the method characterized in including:

a step of extracting a mixture of the pulp fibers, the super absorbent polymers, and water from the used absorbent article,
a step of inactivating the super absorbent polymers by applying voltage to the mixture of the pulp fibers, the super absorbent polymers, and the water by using a pair of electrodes, and
a step of separating the pulp fibers from a mixture of the pulp fibers, inactivated super absorbent polymers, and the water.

The present invention includes the following aspects.

[1] A method of recovering pulp fibers from a used absorbent article which includes the pulp fibers and super absorbent polymers, the method comprising:

a step of extracting a mixture of the pulp fibers, the super absorbent polymers, and water from the used absorbent article,

a step of inactivating the super absorbent polymers by applying voltage to the mixture of the pulp fibers, the super absorbent polymers, and the water by using a pair of electrodes, and

a step of separating the pulp fibers from a mixture of the pulp fibers, inactivated super absorbent polymers, and the water; wherein

the super absorbent polymers are acrylic acid-type, starch-type, or amino acid-type, particulate or fibrous super absorbent polymers.

[2] The method according to [1], further comprising a step of adding water to the used absorbent article before the step of extracting the mixture of the pulp fibers, the super absorbent polymers, and the water from the used absorbent article.

[3] The method according to [1] or [2], further comprising a step of recovering a waste liquid which includes a urine - derived component which is discharged from the super absorbent polymers in the step of inactivating the super absorbent polymers.

[4] The method according to any one of [1] to [3], further comprising a step of recovering a waste liquid which includes a urine - derived component by filtering or dehydrating a residue after the mixture of the pulp fibers, the super absorbent polymers, and the water is extracted from the used absorbent article.

[5] The method according to any one of [1] to [4], further comprising a step of recovering a waste liquid which includes a urine - derived component by further dehydrating the mixture of the pulp fibers, the inactivated super absorbent polymers, and the water, after the step of inactivating the super absorbent polymers and before the step of separating the pulp fibers.

[6] The method according to any one of [3] to [5], further comprising a nutrient salt recovering step of recovering a urine - derived nutrient salt from the waste liquid which includes the urine - derived component.

[7] The method according to any one of [3] to [6], further comprising a microbial fuel cell step of throwing the waste liquid which includes the urine - derived component into a microbial fuel cell so as to reduce a TOC concentration while discharging the water and to recover electric power obtained by power generation.

[8] The method according to any one of [1] to [7], further comprising a sterilizing step of sterilizing the mixture of the pulp fibers, the inactivated super absorbent polymers, and the water, before the step of separating the pulp fibers.

[9] The method according to [8], wherein
the number of viable bacteria in the mixture after the sterilizing step is or less than $1 \times 10^3$.

[10] The method according to any one of [2] to [9], wherein
the step of adding the water to the used absorbent article is a step of immersing the used absorbent article in warm water of 50 °C or higher and lower than 100 °C.

[11] The method according to any one of [2] to [10], wherein
in the step of adding the water to the used absorbent article, a weight of the used absorbent article after being added with the water is or more than 90 % of a maximum absorption weight of the used absorbent article.

[12] The method according to any one of [3] to [11], wherein
the step of extracting the mixture of the pulp fibers, the super absorbent polymers, and the water from the used absorbent article is a step of squeezing out the mixture of the pulp fibers, the super absorbent polymers, and the water from an outer wrapping body of the used absorbent article by letting the used absorbent article pass through a pair of rollers.

[13] The method according to any one of [1] to [12], wherein
the step of separating the pulp fibers is a step of separating the pulp fibers from the inactivated super absorbent polymers by letting the pulp fibers float in the water and precipitating the inactivated super absorbent polymers.

[14] The method according to any one of [1] to [13], further comprising a step of converting a residue after the mixture of the pulp fibers, the super absorbent polymers, and the water is extracted from the used absorbent article or the inactivated super absorbent polymers, to a solid fuel.

[EFFECTS OF THE INVENTION]

In the present invention, the super absorbent polymers are inactivated, desalted, and dehydrated by voltage application, whereby there is no need to use chemicals which deteriorate properties (the ash deposition amount), for the inactivation of the super absorbent polymers. Further, since the cell membranes of bacteria are destroyed and sterilized by voltage application, there is no need to use chemicals which deteriorate pulp fibers, for sterilization. In other words, according to the present invention, since chemicals are not used, there is no deterioration in the properties of pulp fibers (decrease in molecular weight, fiber breakage), and pulp fibers with high safety can be efficiently recovered.

BRIEF DESCRIPTION OF DRAWINGS

[FIG. 1] FIG. 1 is a schematic diagram of one example of an apparatus so as to perform the method of the present invention.
[FIG. 2] FIG. 2 is a diagram for explaining a measurement method of a maximum absorption weight.
[FIG. 3] FIG. 3 shows one example of a configuration of a microbial fuel cell.

MODE FOR CARRYING OUT THE INVENTION

The present invention relates to a method of recovering pulp fibers from a used absorbent article which includes the pulp fibers and super absorbent polymers.
The absorbent article is not particularly limited as long as the absorbent article includes pulp fibers and super absorbent polymers, and a disposable diaper, an incontinence pad, a urine absorbing pad, a sanitary napkin, a panty liner, etc., may be exemplified. In particular, as the absorbent article, an incontinence pad and a disposable diaper which are recovered collectively at facilities, etc., arc preferable since there is no labor for sorting and the amount of pulp is relatively large.
As pulp fibers, although not particularly limited, fluff - like pulp fibers, chemical pulp fibers, etc., may be exemplified.
In the present description, pulp fibers which are recovered according to the method of the present invention is referred to as "recycled pulp".
A super absorbent polymer is also referred to as SAP (Superabsorbent Polymer), which has a three dimensional network structure in which water soluble polymers are moderately crosslinked, is essentially water insoluble although absorbing several ten times to several hundred times of water, and has a function of not releasing water once absorbed even when some pressure is applied. The super absorbent polymer is an acrylic acid type, a starch type, or an amino acid type, particulate or fibrous super absorbent polymer. In the present invention, acrylic acid type super absorbent polymers are preferable from the viewpoint that an effect of lowering the pH by being converted to polyacrylic acid by the inactivation can be expected, and deterioration caused by the pulp fibers being converted to alkali cellulose can be prevented. The acrylic acid type super absorbent polymers have a sodium-substituted carboxyl group - $COONa$, and when water is absorbed, - $COONa$ ionizes to - $COO^-$ and $Na^+$, and dissociates the $Na^+$ ion, the dissociated ion concentration in the super absorbent polymers is increased, water outside the super absorbent polymers enters into the super absorbent polymers by the osmotic pressure difference between inside and outside of the super absorbent polymers, and as a result, the super absorbent polymers swell and retain a large amount of water.
The method according to the present invention includes a step of extracting a mixture of the pulp fibers, super absorbent polymers, and water from a used absorbent article (hereinbelow, which is also referred to simply as "an extracting step"). The method of extracting the mixture of the pulp fibers, the super absorbent polymers, and the water from the used absorbent article is, although not limited thereto, preferably a method of squeezing out the mixture of the pulp fibers, the super absorbent polymers, and the water from an outer wrapping body (a nonwoven fabric, a film, rubber, etc.) of the used absorbent article, by letting the used absorbent article pass through a pair of rollers. In such a case, before letting the used absorbent article pass through a pair of rollers, in order to facilitate squeezing out of the mixture of the pulp fibers, the super absorbent polymers, and the water from the outer wrapping body, the used absorbent article may be let to pass through a pair of rolls for the purpose of crushing and perforating the outer wrapping body of the used absorbent article.
The method according to the present invention includes a step of inactivating the super absorbent polymers by applying voltage to the mixture of the pulp fibers, the super absorbent polymers, and the water by using a pair of electrodes (hereinbelow, which is also referred to simply as "a voltage applying step"). In a case in which the super absorbent polymers include a sodium - substituted carboxyl group (- $COO\ Na^+$), in this step, since the $Na^+$ ions in the super absorbent polymers move toward the minus electrode by electrophoresis due to an electric field formed by the voltage application, $Na^+$ ions are withdrawn from the super absorbent polymers, and the dissociated ion concentration in the super absorbent polymers decreases, whereby the water in the super absorbent polymers goes outside due to the osmotic pressure difference, and the super absorbent polymers dehydrate and contract. The - $COO$ in the super absorbent polymers combines with $H^+$ ions generated by the ionization of the water so as to form - $COOH$, however, since the structure thereof can no longer expand the mesh therein due to the internal crosslinking by the hydrogen bonding being too strong, the super absorbent polymers are to be inactivated.

As the method of applying voltage, although not limited thereto, for example, the mixture of the pulp fibers, the super absorbent polymers, and the water may be sandwiched between horizontally arranged two pieces of wire gauze, and voltage may be applied between the two pieces of wire gauze. The applied voltage is not limited as long as the super absorbent polymers can be inactivated.

By the application of voltage, the waste liquid which is discharged from the super absorbent polymers passes through the wire gauze and falls under the wire gauze by gravity. The waste liquid which has fallen under the wire gauze is recovered in the waste liquid recovery - dedicated container which is disposed under the wire gauze. In the waste liquid, $Na^+$ ions which are withdrawn from the super absorbent polymers, OH ions which are generated by the ionization of the water, urine - derived salts, excrement - derived organic matter, etc., are included.

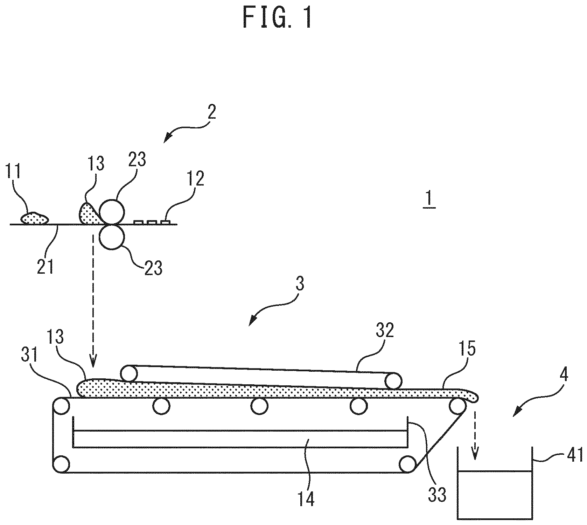

The voltage applying step may be performed by a batch type or in a flow type. When being performed by the flow type, for example, the voltage applying step may be performed by using the apparatus as shown in FIG. 1.

When the inside of the treatment tank is to be under high pH (12.4) environment by lime, cellulose is to be swollen, and pulp fibers are to be converted to alkali cellulose and deteriorated, however, since the method according to the present invention uses the voltage application treatment for the inactivation of the super absorbent polymers, the pH does not change excessively, whereby the pulp fibers would not be deteriorated.

Even when the pulp fibers are regenerated from used diapers, since the deterioration can be suppressed, it is possible to minimize the decrease in quality even when the pulp fibers are repeatedly regenerated.

The method according to the present invention includes a step of separating the pulp fibers from the mixture of the pulp fibers, inactivated super absorbent polymers, and the water (hereinbelow, which is also referred to simply as "a separating step").

The step of separating the pulp fibers from the mixture of the pulp fibers, the inactivated super absorbent polymers, and the water is not limited, however, the separating step is preferably a step of separating the pulp fibers from the inactivated super absorbent polymers by letting the pulp fibers float in the water and precipitating the inactivated super absorbent polymers. Since the inactivated super absorbent polymers have larger specific gravity than the pulp fibers, when the pulp fibers and the inactivated super absorbent polymers are placed in water, the pulp fibers and the inactivated super absorbent polymers are to be separated according to the specific gravity difference, and the pulp fibers which have lighter specific gravity float, and the inactivated super absorbent polymers precipitate, whereby the floated pulp fibers are scooped and recovered.

The method according to the present invention may further include a step of adding the water to the used absorbent article before the step of extracting the mixture of the pulp fibers, the super absorbent polymers, and the water from the used absorbent article (hereinbelow, which is also referred to simply as "a water adding step"). By adding water to the used absorbent article and making the super absorbent polymers sufficiently swollen, it is easier for the pulp fibers and the super absorbent polymers to be extracted from the used absorbent article, and it is possible to reduce the contact resistance between the mixture of the pulp fibers, the super absorbent polymers, and the water and the electrode in the voltage applying step, and further, it is easier for the electricity to flow in the mixture of the pulp fibers, the super absorbent polymers, and the water by the existence of the water, whereby the efficiency of the voltage application treatment is increased. In an absorbent article such as a paper diaper, etc., normally, since an absorbent body which is composed of pulp fibers and super absorbent polymers are sandwiched by upper and lower cover layers (which are the outer wrapping body), it is easy for the absorbent body to be pushed out. Further, by adding water, it is easier for the pulp fibers and the super absorbent polymers to be extracted from the used absorbent article, and as a result, there is little loss in the amount of the pulp fibers to be recovered, whereby the pulp fibers can be recovered efficiently.

The amount of the water to be added is not limited as long as the super absorbent polymers can be inactivated in the voltage applying step, however, the weight of the used absorbent article after being added with the water preferably is or more than 90 % of the maximum absorption weight of the used absorbent article. When the used absorbent article is swollen so that the weight thereof is or more than 90 % of the maximum absorption weight of the used absorbent article, the used absorbent article is to be greatly inflated, whereby it is easy to push out the absorbent body which is composed of pulp fibers and super absorbent polymers, and the absorbent body can be extracted efficiently.

In the present description, the maximum absorption weight corresponds to the weight after the absorbent article is immersed in tap water, according to the following procedure. [The measurement method of the maximum absorption weight]

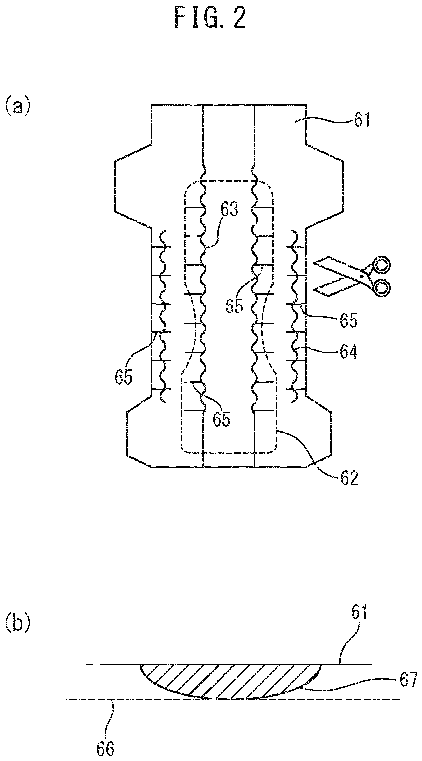

(1) As shown in FIG. 2(a), for the absorbent article 61, notches 65 are formed in the extendable - shrinkable materials 63, 64 which can form pockets so as not to reach the absorbent body 62, and the absorbent article 61 is flattened.
(2) The absorbent article 61 is immersed in a water bath filled with sufficient amount of tap water, with the absorption surface facing downwards, and is left for 30 minutes.
(3) After being left, the absorbent article 61 is placed on the mesh 66 with the absorption surface 67 facing downwards, and the weight thereof after being subjected to draining for 20 minutes is regarded as the maximum absorption weight (refer to FIG. 2(b)).

The method of adding the water is, although not limited thereto, preferably immersing the used absorbent article in the water. According to the method of immersing the used absorbent article in the water, the super absorbent polymers can be swollen, and at the same time, be washed. In a case in which the used absorbent article includes contaminants such as feces, etc., the contaminants such as feces, etc. can also be removed.

The temperature of the water is, although not limited thereto, preferably 55 °C or higher and lower than 100 °C, more preferably 60 °C or higher and lower than 100 °C, and even more preferably 70 °C or higher and lower than 100 °C. By using warm water of 55 °C or higher, the water absorption efficiency of the super absorbent polymers can be increased, a part of bacteria is subjected to primary sterilization, and the hot melt adhesive agent which is used in the absorbent article is softened so that it is easier for the absorbent body to be pushed out, whereby the recovery efficiency of the pulp fibers is increased.

The immersing time is not limited as long as the super absorbent polymers can be inactivated in the voltage applying step, however, is preferably 1 minute or more, is more preferably 5 minutes or more, and even more preferably 10 minutes or more.

The method according to the present invention may further include a step of recovering a waste liquid which includes a urine - derived component which is discharged from the super absorbent polymers in the step of inactivating the super absorbent polymers (hereinbelow, which is also referred to simply as "a waste liquid recovering step"). In the waste liquid, $Na^+$ ions which are withdrawn from the super absorbent polymers, OH ions which are generated by the ionization of the water, urine - derived salts, excrement - derived organic matter, etc., are included. This step can be performed at the same time as the inactivating step. The recovered waste liquid is sent to a nutrient salt recovering step and / or a microbial fuel cell step which are described later and can be used effectively. By recovering and reusing materials other than the pulp fibers, the recycling rate of the used absorbent article is increased.

The method according to the present invention may further include a step of recovering a waste liquid which includes a urine - derived component by filtering or dehydrating a residue after the mixture of the pulp fibers, the super absorbent polymers, and the water is extracted from the used absorbent article (hereinbelow, which is also referred to simply as "a residue filtering / dehydrating step"). In the waste liquid which is recovered by filtering or dehydrating the residue, urine - derived salts, excrement - derived organic matter, etc., are included. The recovered waste liquid is sent to the nutrient salt recovering step and / or a microbial fuel cell step which are described later and can be used effectively. By recovering and reusing materials other than the pulp fibers, the recycling rate of the used absorbent article is increased.

The method according to the present invention may further include a step of recovering a waste liquid which includes a urine - derived component by further dehydrating the mixture of the pulp fibers, the inactivated super absorbent polymers, and the water, after the step of inactivating the super absorbent polymers and before the step of separating the pulp fibers (hereinbelow, which is also referred to simply as "a mixture dehydrating step"). In the waste liquid which is recovered by dehydrating the mixture, urine - derived salts, excrement - derived organic matter, etc., are included. The recovered waste liquid is sent to the nutrient salt recovering step and / or a microbial fuel cell step which are described later and can be used effectively. By recovering and reusing materials other than the pulp fibers, the recycling rate of the used absorbent article is increased.

The method of dehydrating the residue after the mixture of the pulp fibers, the super absorbent polymers, and the water is extracted from the used absorbent article, or of dehydrating the mixture of the pulp fibers, the inactivated super absorbent polymers, and the water is not particularly limited, and roll pressing, belt pressing, screw pressing, etc., can be exemplified.

The method according to the present invention may further include a nutrient salt recovering step of recovering a urine - derived nutrient salt from the waste liquid which includes the urine - derived component. The nutrient salt is salt which includes nitrogen, phosphorus, or potassium, applicable as a fertilizer, and more specifically, ammonium salt, phosphate, etc., may be mentioned. The recovered nutrient salt can be used as a fertilizer.

As the method of recovering the nutrient salt, although not limited thereto, a method of recovering nutrient salt which includes phosphorus by crystallizing phosphorus in the waste liquid as hydroxyapatite (hereinbelow, which is also referred to as "the HAP method"), and a method of recovering nutrient salt which includes phosphorus and / or nitrogen by crystallizing phosphorus and / or nitrogen in the waste liquid as magnesium ammonium phosphate (hereinbelow, which is also referred to as "the MAP method").

The HAP method is a method which uses the crystallization phenomenon of hydroxyapatite ($Ca_{10}(OH)_2(PO_4)_6$) generated by the reaction of $PO_4^{3-}$, $Ca^{2+}$, and OH in the waste liquid. The reaction formula is as follows.

$$10\ Ca^{2+} + 2\ OH^- + 6\ PO_4^{3-} \rightarrow Ca_{10}(OH)_2(PO_4)_6 \qquad (1)$$

In the HAP method, $Ca^{2+}$ and OH are added to an aqueous solution which includes phosphorus, so as to be in contact with the seed crystal in a supersaturated state (metastable region), whereby hydroxyapatite is crystalized on the seed crystal surface and phosphorous in the waste liquid is recovered. As the seed crystal, phosphorous ore, bone charcoal, calcium silicate hydrate, etc., can be used.

In this method, the concentration of $Ca^{2+}$ of 5 millimol / liter or more, pH of 8 or higher, and preferably, the concentration of $Ca^{2+}$ of 10 millimol / liter or more, pH of 9 or higher is required.

The MAP method is a method which uses the crystallization phenomenon of magnesium ammonium phosphate ($MgNH_4PO_4 \cdot 6H_2O$) generated by the reaction of $PO_4^{3-}$, $NH^{4+}$, and $Mg^{2+}$ in the waste liquid. The reaction formula is as follows.

$$Mg^{2+} + NH^{4+} + PO_4^{3-} + 6H_2O \rightarrow MgNH_4PO_4 \cdot 6H_2O \qquad (2)$$

In this method, the concentration of $Mg^{2+}$ of 30 to 60 millimol / liter is preferable, and pH of 6.8 to 7.7 is preferable.

The method according to the present invention may further include a microbial fuel cell step of throwing the waste liquid which includes the urine - derived component into a microbial fuel cell so as to reduce a TOC concentration while discharging the water and to recover electric power obtained by power generation.

In the present description, a microbial fuel cell is a device which converts organic matter as fuel into electric energy by using microorganisms. In the microbial fuel cell, a negative electrode and a positive electrode are immersed in a solution of organic matter which is fuel, electrons generated when the organic matter is oxidatively decomposed by microorganisms are recovered at the negative electrode, the electrons move to the positive electrode via an external circuit, and the electrons are consumed by the reduction reaction of an oxidizing agent at the positive electrode. Electrons flow by the difference of oxidation - reduction potentials between the chemical reaction caused at the negative electrode and the chemical reaction caused at the positive electrode, and energy which corresponds to the product of the potential difference between both poles and the current which flows through the external circuit can be obtained at the external circuit.

In the microbial fuel cell step, the waste liquid is thrown into the microbial fuel cell so as to reduce the TOC concentration while discharging the water, and the electric power obtained by power generation is recovered. In the microbial fuel cell, microorganisms oxidatively decompose organic matter such as dirt, fine pulp, etc., which is included in the waste liquid, whereby the TOC concentration while discharging the water is reduced, and the power generation is performed.

The microorganisms to be used for the microbial fuel cell are not particularly limited, as long as the microorganisms oxidatively decompose organic matter and contribute to generation of electric energy, however, as the microorganisms to be used for the microbial fuel cell, hydrogen producing microorganisms are mainly used, and among which obligate anaerobic bacteria and facultative anaerobic bacteria are preferably used.

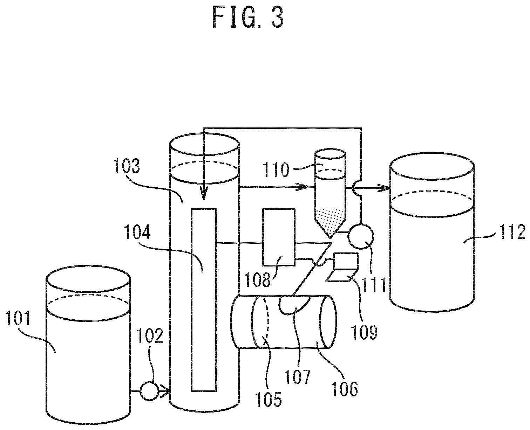

One example of the configuration of the microbial fuel cell is shown in FIG. 3. In the figure, 101 shows the waste liquid tank, 102 shows the pump, 103 shows the negative electrode reaction tank, 104 shows the negative electrode, 105 shows the proton exchange membrane, 106 shows the positive electrode tank, 107 shows the positive electrode, 108 shows the tester, 109 shows the personal computer, 110 shows the sludge precipitation tank, 111 shows the pump, and 112 shows the purification tank.

The pH of the discharged water from the microbial fuel cell step is preferably less than 8.0. When the pH of the discharged water from the microbial fuel cell step is too high, the power generation efficiency in the microbial fuel cell step is decreased.

The TOC concentration of the discharged water from the microbial fuel cell step is preferably 2000 mg / L or lower. When the TOC concentration of the discharged water from the microbial fuel cell step is 2000 mg / L or lower, it is possible to perform purification treatment simply by a general purification tank, etc., in the subsequent step. Further, in a case in which the draining is directly performed from the microbial fuel cell step, the TOC concentration of the discharged water is preferably 30 mg / L or lower.

The method according to the present invention may further include a sterilizing step of sterilizing the mixture of the pulp fibers, the inactivated super absorbent polymers, and the water, before the step of separating the pulp fibers.

According to the method of the present invention, in the voltage applying step, the cell membrane of the bacteria is destroyed and the bacteria are killed by the voltage application, whereby the voltage applying step also has a sterilizing function, and also serves as the sterilizing step. Accordingly, although the method does not necessarily have to be provided with the sterilizing step other than the voltage applying step, in a case in which pulp fibers with higher safety is required, the sterilizing step may be provided under condition ranges which do not deteriorate the properties of the pulp fibers.

The method of the sterilization is preferably not a chemical treatment, and a sterilizing method which does not leave residues such as a heat treatment, electricity, ultraviolet rays, ozone, etc., is preferable.

The number of viable bacteria in the mixture of the pulp fibers, the inactivated super absorbent polymers, and the water after the sterilizing step preferably is or less than $1 \times 10^3$. When the number of viable bacteria is or less than $1 \times 10^3$, pulp fibers with high safety can be obtained.

The method according to the present invention may further include a step of converting a residue after the mixture of the pulp fibers, the super absorbent polymers, and the water is extracted from the used absorbent article or the inactivated super absorbent polymers, to a solid fuel (hereinbelow, which is also referred to simply as "a solid fuel converting step"). In the residue after the mixture of the pulp fibers, the super absorbent polymers, and the water is extracted from the used absorbent article, a nonwoven fabric, a plastic film, rubber, etc., are included. The plastic materials recovered from the used absorbent article can be converted to solid fuel, whereby the plastic materials can be recycled. By recovering and reusing materials other than the pulp fibers, the recycling rate of the used absorbent article is increased. The solid fuel conversion can be performed by so-called RPF conversion technique.

The method according to the present invention may further include a step of washing the separated pulp fibers (hereinbelow, which is also referred to simply as "a pulp fiber washing step").

As the method of washing the separated pulp fibers, although not limited thereto, for example, placing the separated pulp fibers in a mesh bag and rinsing the same with water may be mentioned. The rinsing can be performed in a batch system, in a semi - bath system, or in a circulation system. In a case in which the rinsing is performed in a batch system, for example, the rinsing may be performed by using a washing machine.

The condition of the washing is not particularly limited as long as substances other than the pulp fibers are sufficiently removed, and for example, the washing time is preferably 3 to 60 minutes, more preferably 5 to 50 minutes, and even more preferably 10 to 40 minutes. In a case in which the rinsing is performed in a batch system, the amount of the water to be used with respect to 100 parts by mass (absolute dry mass) of the pulp fibers is preferably 500 to 5000 parts by mass, is more preferably 800 to 4000 parts by mass, and even more preferably 1000 to 3000 parts by mass.

The method according to the present invention may further include a step of dehydrating the washed pulp fibers (hereinbelow, which is also referred to as "a pulp fiber dehydrating step").

As the method of dehydrating the washed pulp fibers, although not limited thereto, for example, dehydrating the washed pulp fibers which are placed in a mesh bag by a dehydrating machine may be mentioned.

The condition of the dehydrating is not particularly limited as long as the moisture rate can be lowered to the target value, and for example, the dehydrating time is preferably 1 to 10 minutes, and is more preferably 2 to 8 minutes.

The method according to the present invention may further include a step of drying the dehydrated pulp fibers (hereinbelow, which is also referred to as "a pulp fiber drying step").

As the method of drying the dehydrated pulp fibers, although not limited thereto, for example, drying the dehydrated pulp fibers by using a drying machine such as a hot air drying machine, etc., may be mentioned.

The condition of the drying is not particularly limited as long as the pulp fibers are sufficiently dried, and for example, the drying temperature is preferably 100 to 200 °C, more preferably 110 to 180 °C, and even more preferably 120 to 160 °C. The drying time is preferably 10 to 120 minutes, more preferably 20 to 80 minutes, and even more preferably 30 to 60 minutes.

The moisture rate of the pulp fibers after being dried is preferably 5 to 13 %, more preferably 6 to 12 %, and even more preferably 7 to 11 %. When the moisture rate is too low, there may be cases in which the hydrogen bonding is stronger and the pulp fibers are to be too stiff, and on the contrary, when the moisture rate is too high, there may be cases in which fungi, etc., occur.

The moisture rate of the pulp fibers is measured as follows. Incidentally, this measurement is performed under the atmosphere of 20 °C ± 1 °C.

(1) The mass A (g) of the container (without a lid) in which the measurement target sample is placed is measured.
(2) Approximately 5 g of the measurement target sample is prepared, is placed in the container which has been subjected to the mass measurement in (1), and the mass B (g) of the container in which the sample is placed is measured.
(3) The container in which the sample is placed is left in an oven in which the temperature is 105 °C ± 3 °C for 2 hours.
(4) The container in which the sample is placed is removed from the oven, and is left in a desiccator (in which a desiccant: colored silica gel is placed) for 30 minutes.
(5) The container in which the sample is placed is removed from the desiccator, and the mass C (g) is measured.
(6) The moisture rate (5) is calculated by the following formula.

$$\text{Moisture rate } (\%) = (B - C) / (C - A) \times 100$$

Hereinbelow, the present invention is further explained with reference to the drawings, however, the present invention is not limited to the embodiment shown in the drawings.

FIG. 1 is a schematic diagram of one example of the apparatus 1 so as to perform the present invention.

The apparatus 1 is configured by including an extracting step 2, a voltage applying step 3, and a separating step 4.

The extracting step 2 includes a conveyor 21 and a pair of rollers 23. The used absorbent article 11 is conveyed while being placed on the conveyor 21 so as to be sent to the pair of rollers 23. At this time, the outer wrapping body

# EP 3 417 954 B1

12 which configures the used absorbent article passes through between the pair of rollers 23, the mixture of the pulp fibers, the super absorbent polymers, and the water is squeezed out from the outer wrapping body by the pair of rollers 23, so as to stay before the pair of rollers 23. The mixture of the pulp fibers, the super absorbent polymers, and the water which stays before the pair of rollers 23 is regularly raked out, so as to be sent to the voltage applying step 3.

The voltage applying step 3 includes the first electrode 31 and the second electrode 32. The second electrode 32 is provided above the first electrode 31. The first electrode 31 is a belt conveyor made of wire mesh. The second electrode 32 is also made into a belt - like shape, and only has to be made of an electrically conductive material, and does not necessarily has to be made of wire mesh, although the second electrode 32 may be made of wire mesh. A predetermined voltage is applied between the first electrode 31 and the second electrode 32 (which is not shown). Either the first electrode 31 or the second electrode 32 may be the positive electrode. The lower half of the belt which configures the second electrode 32 moves at the same speed (from left to right in the drawing) as the upper half of the belt which configures the first electrode 31. The gap between the first electrode 31 and the second electrode 32 is set so as to be able to be adjusted, and preferably, the gap is adjusted so that the gap is narrowed as moving from left to right. The mixture of the pulp fibers, the super absorbent polymers, and the water 13 which is extracted from the used absorbent article in the extracting step is conveyed while being placed on the first electrode 31, and is sandwiched between the first electrode 31 and the second electrode 32, so as to be applied with voltage. From the super absorbent polymers which have been applied with voltage, waste liquid 14 which includes $Na^+$ ions, $OH^-$ ions, urine - derived salts, excrement - derived organic matter, etc., is discharged. The discharged waste liquid 14 passes through the wire mesh which configures the first electrode 31, drops into the waste liquid recovery - dedicated container 33 which is provided under the first electrode 31, and is stored therein. From the exit port of the voltage applying step 3, the mixture of the pulp fibers, the inactivated super absorbent polymers, and the water 15 is discharged, so as to be sent to the separating step 4.

The separating step 4 includes the separation tank 41. In the separation tank 41, the mixture of the pulp fibers, the inactivated super absorbent polymers, and the water 15 which has been sent from the voltage applying step 3 is thrown. Additional water is thrown into the separation tank 41, and is stirred and left still, whereby since the inactivated super absorbent polymers have larger specific gravity than the pulp fibers, the inactivated super absorbent polymers are precipitated in the bottom of the separation tank 41, and the pulp fibers float. The floating pulp fibers are scooped.

INDUSTRIAL APPLICABILITY

The method of the present invention can be preferably used for recycling a used absorbent article, such as a paper diaper, etc.

REFERENCE SIGNS LIST

1 apparatus
2 extracting step
3 voltage applying step
4 saccharifying step
11 used absorbent article
12 outer wrapping body
13 mixture of pulp fibers, super absorbent polymers, and water
14 waste liquid
15 mixture of pulp fibers, inactivated super absorbent polymers, and water
21 conveyor
23 pair of rollers
31 first electrode
32 second electrode
33 waste liquid recovery - dedicated container
41 separation tank
101 waste liquid tank
102 pump
103 negative electrode reaction tank
104 negative electrode
105 proton exchange membrane
106 positive electrode tank
107 positive electrode

9

108    tester
109    personal computer
110    sludge precipitation tank
111    pump
112    purification tank

**Claims**

1.  A method of recovering pulp fibers from a used absorbent article which includes the pulp fibers and super absorbent polymers, the method comprising:

    a step of extracting a mixture of the pulp fibers, the super absorbent polymers, and water from the used absorbent article,
    a step of inactivating the super absorbent polymers by applying voltage to the mixture of the pulp fibers, the super absorbent polymers, and the water by using a pair of electrodes, and
    a step of separating the pulp fibers from a mixture of the pulp fibers, inactivated super absorbent polymers, and the water; wherein
    the super absorbent polymers are acrylic acid-type, starch-type, or amino acid-type, particulate or fibrous super absorbent polymers.

2.  The method according to claim 1, further comprising a step of adding water to the used absorbent article before the step of extracting the mixture of the pulp fibers, the super absorbent polymers, and the water from the used absorbent article.

3.  The method according to claim 1 or 2, further comprising a step of recovering a waste liquid which includes a urine - derived component which is discharged from the super absorbent polymers in the step of inactivating the super absorbent polymers.

4.  The method according to any one of claims 1 to 3, further comprising a step of recovering a waste liquid which includes a urine - derived component by filtering or dehydrating a residue after the mixture of the pulp fibers, the super absorbent polymers, and the water is extracted from the used absorbent article.

5.  The method according to any one of claims 1 to 4, further comprising a step of recovering a waste liquid which includes a urine - derived component by further dehydrating the mixture of the pulp fibers, the inactivated super absorbent polymers, and the water, after the step of inactivating the super absorbent polymers and before the step of separating the pulp fibers.

6.  The method according to any one of claims 3 to 5, further comprising a nutrient salt recovering step of recovering a urine - derived nutrient salt from the waste liquid which includes the urine - derived component.

7.  The method according to any one of claims 3 to 6, further comprising a microbial fuel cell step of throwing the waste liquid which includes the urine - derived component into a microbial fuel cell so as to reduce a TOC concentration while discharging the water and to recover electric power obtained by power generation.

8.  The method according to any one of claims 1 to 7, further comprising a sterilizing step of sterilizing the mixture of the pulp fibers, the inactivated super absorbent polymers, and the water, before the step of separating the pulp fibers.

9.  The method according to claim 8, wherein
    the number of viable bacteria in the mixture after the sterilizing step is or less than $1 \times 10^3$.

10. The method according to any one of claims 2 to 9, wherein
    the step of adding the water to the used absorbent article is a step of immersing the used absorbent article in warm water of 50 °C or higher and lower than 100 °C.

11. The method according to any one of claims 2 to 10, wherein
    in the step of adding the water to the used absorbent article, a weight of the used absorbent article after being added with the water is or more than 90 % of a maximum absorption weight of the used absorbent article.

12. The method according to any one of claims 3 to 11, wherein
the step of extracting the mixture of the pulp fibers, the super absorbent polymers, and the water from the used absorbent article is a step of squeezing out the mixture of the pulp fibers, the super absorbent polymers, and the water from an outer wrapping body of the used absorbent article by letting the used absorbent article pass through a pair of rollers.

13. The method according to any one of claims 1 to 12, wherein
the step of separating the pulp fibers is a step of separating the pulp fibers from the inactivated super absorbent polymers by letting the pulp fibers float in the water and precipitating the inactivated super absorbent polymers.

14. The method according to any one of claims 1 to 13, further comprising a step of converting a residue after the mixture of the pulp fibers, the super absorbent polymers, and the water is extracted from the used absorbent article or the inactivated super absorbent polymers, to a solid fuel.

**Patentansprüche**

1. Verfahren für die Rückgewinnung von Zellstofffasern aus einem benutzten absorbierenden Artikel, der die Zellstofffasern und superabsorbierenden Polymere einschließt, wobei das Verfahren Folgendes umfasst:

   einen Schritt des Extrahierens einer Mischung der Zellstofffasern, der superabsorbierenden Polymere und von Wasser aus dem benutzten absorbierenden Artikel,
   einen Schritt des Inaktivierens der superabsorbierenden Polymere durch Anlegen einer Spannung an die Mischung der Zellstofffasern, der superabsorbierenden Polymere und des Wassers durch Verwenden eines Paars von Elektroden und
   einen Schritt des Abtrennens der Zellstofffasern aus einer Mischung der Zellstofffasern, inaktivierten superabsorbierenden Polymere und des Wassers, wobei
   die superabsorbierenden Polymere Acrylsäure-artige, Stärke-artige oder Aminosäure-artige, partikelförmige oder faserförmige superabsorbierende Polymere sind.

2. Verfahren nach Anspruch 1, das weiter einen Schritt des Zugebens von Wasser zu dem benutzten absorbierenden Artikel vor dem Schritt des Extrahierens der Mischung der Zellstofffasern, der superabsorbierenden Polymere und des Wassers aus dem benutzten absorbierenden Artikel umfasst.

3. Verfahren nach Anspruch 1 oder 2, das weiter einen Schritt des Rückgewinnens einer Abfallflüssigkeit umfasst, die eine von Urin stammende Komponente einschließt, die aus den superabsorbierenden Polymeren in dem Schritt des Inaktivierens der superabsorbierenden Polymere abgegeben wird.

4. Verfahren nach einem der Ansprüche 1 bis 3, das weiter einen Schritt des Rückgewinnens einer Abfallflüssigkeit umfasst, die eine von Urin stammende Komponente einschließt, durch Filtern oder Entwässern eines Rests, nachdem die Mischung der Zellstofffasern, der superabsorbierenden Polymere und des Wassers aus dem benutzten absorbierenden Artikel extrahiert ist.

5. Verfahren nach einem der Ansprüche 1 bis 4, das weiter einen Schritt des Rückgewinnens einer Abfallflüssigkeit umfasst, die eine von Urin stammende Komponente einschließt, durch weiteres Entwässern der Mischung der Zellstofffasern, der inaktivierten superabsorbierenden Polymere und des Wassers nach dem Schritt des Inaktivierens der superabsorbierenden Polymere und vor dem Schritt des Abtrennens der Zellstofffasern.

6. Verfahren nach einem der Ansprüche 3 bis 5, das weiter einen Nährsalzrückgewinnungsschritt des Rückgewinnens eines von Urin stammenden Nährsalzes aus der Abfallflüssigkeit umfasst, die die von Urin stammende Komponente einschließt.

7. Verfahren nach einem der Ansprüche 3 bis 6, das weiter einen Schritt mit mikrobieller Brennstoffzelle des Werfens der Abfallflüssigkeit, die die von Urin stammende Komponente einschließt, in eine mikrobielle Brennstoffzelle umfasst, um eine TOC-Konzentration zu verringern, während das Wasser abgegeben wird, und um elektrische Energie zu gewinnen, die durch Energieerzeugung erhalten wird.

8. Verfahren nach einem der Ansprüche 1 bis 7, das weiter einen Sterilisierungsschritt des Sterilisierens der Mischung der Zellstofffasern, der inaktivierten superabsorbierenden Polymere und des Wassers vor dem Schritt des Abtrennens der Zellstofffasern umfasst.

**9.** Verfahren nach Anspruch 8, wobei
die Anzahl von lebensfähigen Bakterien in der Mischung nach dem Sterilisierungsschritt $1 \times 10^3$ oder weniger beträgt.

**10.** Verfahren nach einem der Ansprüche 2 bis 9, wobei
der Schritt des Zugebens des Wassers zu dem benutzten absorbierenden Artikel ein Schritt des Eintauchens des benutzten absorbierenden Artikels in warmes Wasser von 50 °C oder höher und niedriger als 100 °C ist.

**11.** Verfahren nach einem der Ansprüche 2 bis 10, wobei
in dem Schritt des Zugebens des Wassers zu dem benutzten absorbierenden Artikel ein Gewicht des benutzten absorbierenden Artikels nach dem Zugeben des Wassers 90 % oder mehr eines Maximalabsorptionsgewichts des benutzten absorbierenden Artikels beträgt.

**12.** Verfahren nach einem der Ansprüche 3 bis 11, wobei
der Schritt des Extrahierens der Mischung der Zellstofffasern, der superabsorbierenden Polymere und des Wassers aus dem benutzten absorbierenden Artikel ein Schritt des Auspressens der Mischung der Zellstofffasern, der superabsorbierenden Polymere und des Wassers aus einem äußeren Umhüllungskörper des benutzten absorbierenden Artikels durch das Durchlaufen des benutzten absorbierenden Artikels durch ein Walzenpaar ist.

**13.** Verfahren nach einem der Ansprüche 1 bis 12, wobei
der Schritt des Abtrennens der Zellstofffasern ein Schritt des Abtrennens der Zellstofffasern von den inaktivierten superabsorbierenden Polymeren durch Aufschwimmen der Zellstofffasern auf dem Wasser und Ausfällen der inaktivierten superabsorbierenden Polymere ist.

**14.** Verfahren nach einem der Ansprüche 1 bis 13, das weiter einen Schritt des Umwandeln eines Rests, nachdem die Mischung der Zellstofffasern, der superabsorbierenden Polymere und des Wassers aus dem benutzten absorbierenden Artikel oder den inaktivierten superabsorbierenden Polymeren extrahiert ist, zu einem festen Brennstoff umfasst.

**Revendications**

**1.** Procédé de récupération de fibres de pâte à papier en provenance d'un article absorbant usagé qui comprend les fibres de pâte à papier et des polymères super absorbants, le procédé comportant :

une étape consistant à extraire un mélange constitué par les fibres de pâte à papier, les polymères super absorbants, et de l'eau en provenance de l'article absorbant usagé,
une étape consistant à inactiver les polymères super absorbants en appliquant une tension sur le mélange constitué par les fibres de pâte à papier, les polymères super absorbants, et l'eau en utilisant une paire d'électrodes, et
une étape consistant à séparer les fibres de pâte à papier en provenance d'un mélange constitué par les fibres de pâte à papier, les polymères super absorbants inactivés, et l'eau ; dans lequel
les polymères super absorbants sont des polymères super absorbants en particules ou en fibres du type acide acrylique, du type amidon, ou du type acide aminé.

**2.** Procédé selon la revendication 1, comportant par ailleurs une étape consistant à ajouter de l'eau à l'article absorbant usagé avant l'étape consistant à extraire le mélange constitué par les fibres de pâte à papier, les polymères super absorbants, et l'eau en provenance de l'article absorbant usagé.

**3.** Procédé selon la revendication 1 ou la revendication 2, comportant par ailleurs une étape consistant à récupérer un liquide résiduaire qui comprend un composant dérivé de l'urine qui est déchargé en provenance des polymères super absorbants au cours de l'étape consistant à inactiver les polymères super absorbants.

**4.** Procédé selon l'une quelconque des revendications 1 à 3, comportant par ailleurs une étape consistant à récupérer un liquide résiduaire qui comprend un composant dérivé de l'urine en filtrant ou en déshydratant un résidu après que le mélange constitué par les fibres de pâte à papier, les polymères super absorbants, et l'eau est extrait en provenance de l'article absorbant usagé.

5. Procédé selon l'une quelconque des revendications 1 à 4, comportant par ailleurs une étape consistant à récupérer un liquide résiduaire qui comprend un composant dérivé de l'urine en déshydratant plus encore le mélange constitué par les fibres de pâte à papier, les polymères super absorbants inactivés, et l'eau, après l'étape consistant à inactiver les polymères super absorbants et avant l'étape consistant à séparer les fibres de pâte à papier.

6. Procédé selon l'une quelconque des revendications 3 à 5, comportant par ailleurs une étape de récupération de sel nutritif consistant à récupérer un sel nutritif dérivé de l'urine en provenance du liquide résiduaire qui comprend le composant dérivé de l'urine.

7. Procédé selon l'une quelconque des revendications 3 à 6, comportant par ailleurs une étape de pile à combustible microbienne consistant à jeter le liquide résiduaire qui comprend le composant dérivé de l'urine dans une pile à combustible microbienne de manière à réduire une concentration en TOC tout en déchargeant l'eau et à récupérer la puissance électrique obtenue par la production d'énergie.

8. Procédé selon l'une quelconque des revendications 1 à 7, comportant par ailleurs une étape de stérilisation consistant à stériliser le mélange constitué par les fibres de pâte à papier, les polymères super absorbants inactivés, et l'eau, avant l'étape consistant à séparer les fibres de pâte à papier.

9. Procédé selon la revendication 8, dans lequel
le nombre de bactéries revivifiables dans le mélange après l'étape de stérilisation est égal ou inférieur à $1 \times 10^3$.

10. Procédé selon l'une quelconque des revendications 2 à 9, dans lequel
l'étape consistant à ajouter de l'eau à l'article absorbant usagé est une étape consistant à immerger l'article absorbant usagé dans de l'eau chaude à une température 50 °C ou plus et inférieure à 100 °C.

11. Procédé selon l'une quelconque des revendications 2 à 10, dans lequel
dans l'étape consistant à ajouter de l'eau à l'article absorbant usagé, un poids de l'article absorbant usagé après l'ajout de l'eau est égal ou supérieur à 90 % d'un poids d'absorption maximum de l'article absorbant usagé.

12. Procédé selon l'une quelconque des revendications 3 à 11, dans lequel
l'étape consistant à extraire le mélange constitué par les fibres de pâte à papier, les polymères super absorbants, et l'eau en provenance de l'article absorbant usagé est une étape consistant à faire sortir par pression le mélange constitué par les fibres de pâte à papier, les polymères super absorbants, et l'eau en provenance d'un corps d'emballage extérieur de l'article absorbant usagé en faisant passer l'article absorbant usagé au travers d'une paire de cylindres.

13. Procédé selon l'une quelconque des revendications 1 à 12, dans lequel
l'étape consistant à séparer les fibres de pâte à papier est une étape consistant à séparer les fibres de pâte à papier en provenance des polymères super absorbants inactivés en laissant les fibres de pâte à papier flotter dans l'eau et en faisant précipiter les polymères super absorbants inactivés.

14. Procédé selon l'une quelconque des revendications 1 à 13, comportant par ailleurs une étape consistant à convertir un résidu après que le mélange constitué par les fibres de pâte à papier, les polymères super absorbants, et l'eau est extrait en provenance de l'article absorbant usagé ou des polymères super absorbants inactivés, en un combustible solide.

FIG. 1

# FIG. 2

(a)

(b)

# FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010084031 A
- EP 3156541 A1
- JP 2015134845 A

**Non-patent literature cited in the description**

- **HANAA GADALLAH et al.** Study of Electro De-swelling Properties of Super Absorbents Polymers. *AUSTRALIAN JOURNAL OF BASIC AND APPLIED SCIENCES,* 01 January 2012, vol. 6, 282-291